(19) Europäisches Patentamt
European Patent Office
Office européen des brevets

(11) Publication number : 0 362 119 B1

(12) # EUROPEAN PATENT SPECIFICATION

(45) Date of publication of patent specification : **28.07.93 Bulletin 93/30**

(51) Int. Cl.$^5$ : **C07C 231/02,** C07C 233/44

(21) Application number : **89630161.1**

(22) Date of filing : **22.09.89**

(54) **Improved process for the catalyzed synthesis of N-substituted acrylamides and methacrylamides.**

The file contains technical information submitted after the application was filed and not included in this specification

(30) Priority : **26.09.88 US 249550**

(43) Date of publication of application : **04.04.90 Bulletin 90/14**

(45) Publication of the grant of the patent : **28.07.93 Bulletin 93/30**

(84) Designated Contracting States : **DE FR GB IT**

(56) References cited :
**EP-A- 0 117 530**
**EP-A- 0 216 966**
**GB-A- 2 021 101**
**GB-A- 2 075 495**
**GB-A- 2 100 732**
**US-A- 4 613 673**

(56) References cited :
**PATENT ABSTRACTS OF JAPAN, vol. 12, no. 40 (C-474)(2887), 5 February 1988 & JP-A-62 187 710**
**JOURNAL OF MACROMOLECULAR SCIENCE. REVIEWS IN MACROMOLECULAR CHEMISTRY AND PHYSICS, vol. C22, no.2, 1982/83, pages 169-202, New York, US; M. DAGONNEAU et al.: "Sterically Hindered Amines and Nitroxyls as Polymer Stabilizers"**

(73) Proprietor : **THE GOODYEAR TIRE & RUBBER COMPANY**
**1144 East Market Street**
**Akron, Ohio 44316-0001 (US)**

(72) Inventor : **Parker, Dane Kenton**
**945 Norwich Avenue**
**Massillon Ohio 44646 (US)**
Inventor : **Goodwin, Jeffrey Alan**
**4701 Medina Road**
**Akron Ohio 44321-1163 (US)**

(74) Representative : **Leitz, Paul**
**Goodyear Technical Center-Luxembourg**
**L-7750 Colmar-Berg (LU)**

EP 0 362 119 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a process for producing N-substituted acrylamides or methacrylamides, and more particularly, to a process for the catalyzed synthesis of N-substituted acrylamides or methacrylamides by reacting an acrylic or methacrylic acid ester with an amine in the presence of a polymerization inhibitor containing a hindered nitroxyl group.

Several references in the recent literature, see EP-A-117 530, U.S. Patents 4,206,143 and 4,321,411, have disclosed that various tin derivatives are capable of catalyzing the aminolysis reaction between esters of acrylic or methacrylic acid and amines to produce selective N-substituted acrylamides or methacrylamides. With the use of these catalysts, undesirable Michael-addition, i.e., addition of the amine to the acrylate double bond, is largely avoided. Depending on the particular reactants, reaction temperature, catalyst concentration, etc., these aminolysis reactions may proceed for extended periods (i.e. greater than 10 hours) before high conversions are achieved. Under these conditions, thermal polymerization of the starting (meth)acrylic esters and/or the product N-substituted (meth)acrylamides may occur leading to extensive product separation and clean-up procedures.

JP-A-62 187710 discloses a polymerization inhibitor which can inhibit an N-monoalkyl-(meth)acrylamide from undergoing popcorn polymerization by using a compound selected among nitroso compounds and inorganic and organic stableradicals as a component. The polymerization inhibitor is obtained by using at least one compound selected from among (a) a nitroso compound (e.g., 1-nitroso-2-naphthol), (b) an inorganic stable radical (e.g. NO) and (c) an organic stable radical (e.g. an organic nitroxide radical having vicinol gemdimethyl substituents.

The prior art teaches that to avoid such thermal polymerization side reactions, the aminolysis reaction should be carried out in the presence of a polymerization inhibitor such as 4-methyl-2,6-di-t-butylphenol, monomethyl ether of hydroquinone, etc. in the presence of a little air. Although the use of these inhibitors are known to prevent acrylate polymerization at low temperatures and short reaction times, these inhibitors are inefficient at high temperatures for prolonged periods.
Unfortunately, the prior art does not disclose an efficient method of minimizing such thermal polymerization at high temperatures.

As can be appreciated by those skilled in the art, it would be desirable to have no polymeric or "high boiling impurities" left after separating the product from the tin-catalyst residues. This situation would allow (assuming the catalyst residue is still active) direct recycling of these residues back into the next reaction. Such a situation would offer many advantages including lower catalyst costs, reduced waste disposal and simpler process design. The prior art does not, however, provide any teaching of a method for the significant avoidance or polymeric or high boiling impurities thus allowing the isolation and recycle of catalyst residue. Therefore, there exists a need for a process which substantially minimizes by-product formation and permits the isolation and recycle of catalyst residue.

### Summary of the Invention

The present invention provides a new and improved process for the catalyzed synthesis of N-substituted acrylamide or methacrylamide derivatives by reacting an alkyl ester of acrylic acid or methacrylic acid with an aliphatic or aromatic amine in the presence of a polymerization inhibitor containing a hindered nitroxyl group. The present invention substantially eliminates by-product polymer formation and greatly improves product quality. In addition, the present invention permits substantial cost savings by recycling residues.

### Detailed Description of the Invention

The present invention provides a process that substantially eliminates or minimizes thermal polymerization during the synthesis of N-substituted acrylamide or methacrylamide derivatives. By eliminating the formation of such by-products, one may recycle the catalyst residues and lower the overall cost of the process.

The polymerization inhibitors for use in the present invention contain at least one hindered nitroxyl group. Inclusive of the inhibitors which are suitable for use in the present invention are of the formula:

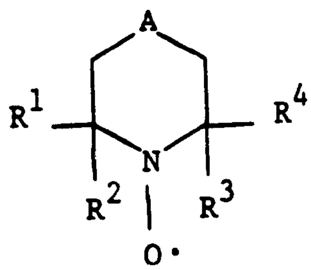

(I),

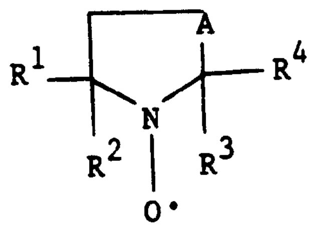

(II),

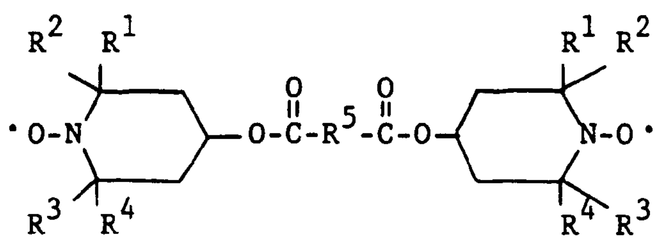

(III),

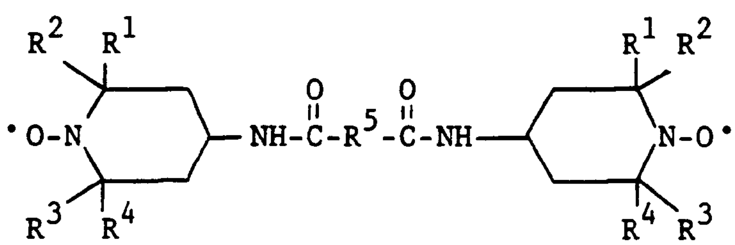

(IV),

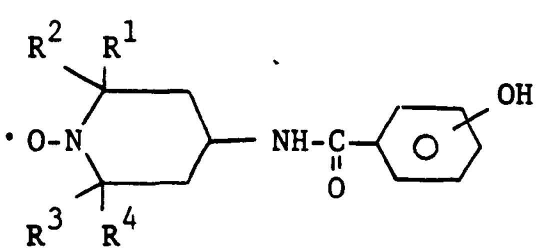

(V),

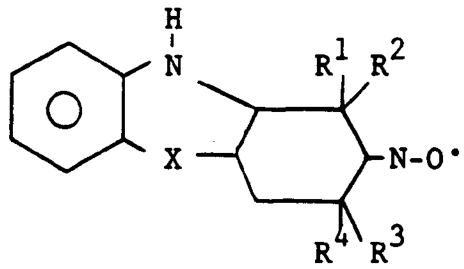

(VI),

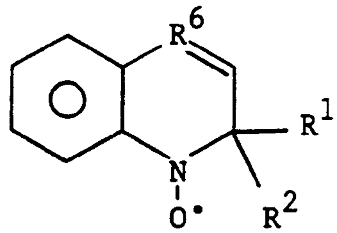

(VII),

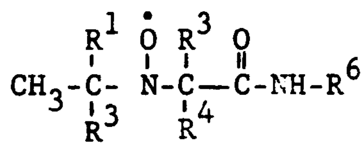

(VIII),

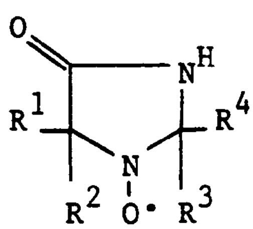

(IX),

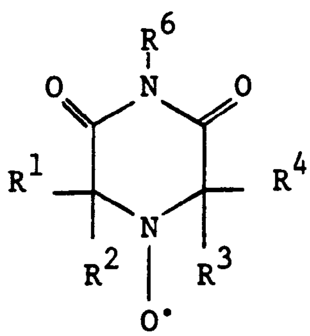

(X),

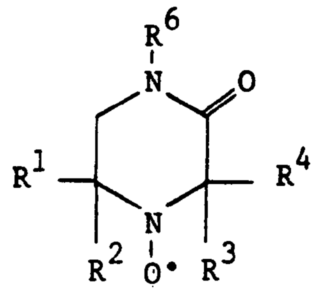

(XI),

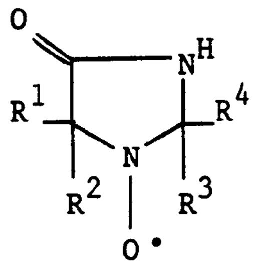

(XII),

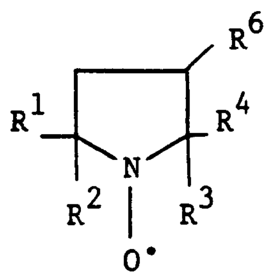

(XIII),

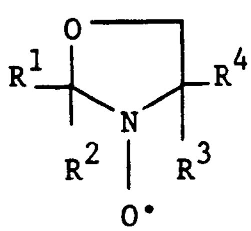

(XIV), and

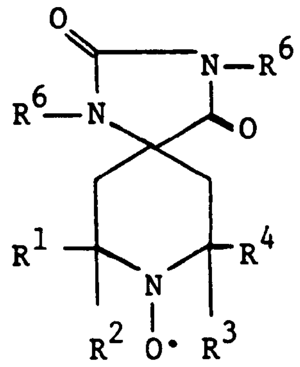

(XV)

wherein $R^1$, $R^2$, $R^3$ and $R^4$ are independently linear or non-alpha branched alkyl radicals having 1 to 9 carbon atoms or one or both of $R^1$ and $R^2$ or $R^3$ and $R^4$ form a cycloalkyl ring having 5 to 7 carbon atoms inclusive of the carbon atom in the hetero ring; wherein A is $-CH_2-$,

$$-\underset{|}{C}=O,\ -\underset{|}{C}H-OR^8,$$

$$-\underset{|}{C}H-O-\overset{O}{\overset{\|}{C}}-R^8,$$

$$-\overset{|}{C}H-NHR^7,\quad -\overset{|}{C}H-NR^7R^8$$

wherein $R^7$ and $R^8$ are independently hydrogen radicals, alkyl radicals having 1 to 18 carbon atoms, aryl radicals having from 6 to 14 carbon atoms or aralkyl radicals having from 7 to 15 carbon atoms; wherein $R^5$ is an alkylene radical having from 5 to 10 carbon atoms, an arylene radical having from 6 to 14 carbon atoms or an aralkylene radical having from 7 to 15 carbon atoms; wherein $R^6$ is independently an alkyl radical having 1 to 18 carbon atoms, an aryl radical having 6 to 14 carbon atoms or an aralkyl radical and wherein x is NH, S or O. Preferred nitroxyl group containing polymerization inhibitors are those of formula I, where $R^1$, $R^2$, $R^3$, and $R^4$ are each a linear alkyl radical having 1 carbon atom and A is $-CH_2-$ or

$$-\underset{|}{C}HOH.$$

Another group of preferred inhibitors are of the formula III where $R^1$, $R^2$, $R^3$ and $R^4$ are each a linear alkyl radical having 1 carbon atom and $R^5$ is an alkylene radical having 8 carbon atoms or an arylene radical having 6 carbon atoms.

Techniques known to those skilled in this art may be used to prepare the inhibitors containing a hindered nitroxyl group. Representative of these methods of preparation are illustrated in J. Keana, "Newer Aspects of the Synthesis and Chemistry of Nitroxides Spin Labels", Chem. Reviews, 78, 37-61 (1978) and E. Rozantsev et al, Dokl. Akad. Nauk SSSR 261, V/1, 109-110 (1981). Each of these references are incorporated by reference in their entirety.

An alkyl ester of acrylic or methacrylic acid is reacted with an aliphatic or aromatic amine in the presence of a catalyst and the inhibitor containing at least one hindered nitroxyl group. The alkyl ester of acrylic or methacrylic acid may be of the formula:

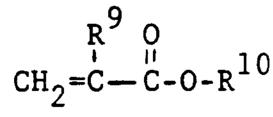

where $R^9$ is a hydrogen radical or methyl radical, and $R^{10}$ is an alkyl radical having from about 1 to 4 carbon atoms. The preferred acrylates or methacrylates are methyl acrylate, ethyl acrylate, methyl methacrylate and ethyl methacrylate since these are readily accessible industrially and the alcohol liberated upon aminolysis can easily be removed from the reaction mixture. As the number of carbon atoms in the alcohol group increases, the suitability of the ester decreases. From this point of view, alkyl esters having less than 4 carbon atoms in the alkyl group are preferred.

The alkyl ester of acrylic or methacrylic acid is reacted with an aliphatic or aromatic amine. Examples of such amines include those of the formula:

$$HNR^{11}R^{12}$$

where $R^{11}$ is a hydrogen radical or alkyl radical having from about 1 to 4 carbon atoms and $R^{12}$ is an alkyl radical having from about 1 to about 20 carbon atoms, aryl radical having from about 6 to 20 carbon atoms, alkaryl radical having from about 7 to 20 carbon atoms, aralkyl radical having from about 7 to about 20 carbon atoms, or alkoxyalkyl radical having from about 2 to about 20 carbon atoms. $R^{12}$ may also be:

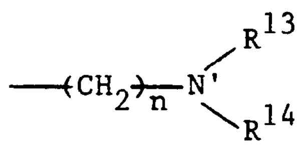

where n is an integer of from 2 to 6 and $R^{13}$ and $R^{14}$ are independently alkyl groups having from about 1 to 4 carbon atoms or $R^{13}$ and $R^{14}$ taken jointly are combined with the N' atom to form a heterocyclic ring group selected from the group consisting of morpholine, pyrrolidine or piperidine ring groups.

Examples of specific amines which would be suitable are aniline, butylamine, n-octylamine, benzylamine, dimethylaminoneopentanamine, dimethylaminoethylamine, 3-dimethyl-aminopropylamine, 3-methylaminopropylamine, 2-dibutylaminoethylamine, 4-(aminopropyl) morpholine, 3-diethylaminopropylamine, hexamethylenediamine, 2-dimethylaminoethylamine, 1-(aminopropyl)piperidine, 4-(aminoethyl)morpholine, and p-aminodiphenylamine.

Stoichiometrically, equimolar amounts of the acrylic or methacrylic acid esters and the amine are needed to form the respective N-substituted acrylamide or methacrylamide. However, an excess of ester may be used. Therefore, the mole ratio of the amine to ester may range from about 1:1 to 1:100. Preferably, the mole ratio of amine to ester ranges from about 1:2 to about 1:5. In order to maintain the respective mole ratio of the reactants, the total amount of reactants may be initially charged or either the amine or ester may be added gradually under the reaction conditions to the reaction mixture containing the other reactant.

While the reaction conditions may vary, they should be such to promote the aminolysis reaction. The reaction between the amine and the ester may be conducted at a temperature in the range of from about 40°C to 200°C. Preferably the reaction is conducted at a temperature ranging from about 70°C to about 150°C. A reaction temperature ranging from about 110°C to 140°C is particularly preferred. Reaction pressure should be atmospheric to subatmospheric. The reaction time will obviously vary depending on such factors as the reaction temperature, reaction pressure, purity of the reactants, reactivity of the catalyst, desired extent of reaction of reactants and amount of catalyst, to name a few. Generally, the reaction may be conducted over a period of from about two to about twenty hours.

The reaction vessel should be equipped with a means of agitation and an inlet for the introduction of an inert gas such as nitrogen. The reaction vessel is preferably equipped with a means of removing the alcohol produced in the reaction. The alcohol may be removed by a suitable distillation column at a suitable reflux ratio.

The aminolysis reaction is conducted in the presence of a catalyst. The catalyst is generally dissolved or suspended in the acrylic or methacrylic acid ester. The catalyst may be an alkyltin alkoxide, dialkyltin alkoxide or trialkyltin alkoxide. Examples of alkyltin alkoxide catalysts are described in U.S. Patent 4,321,114. Suitable alkyltin alkoxides, dialkyltin alkoxides and trialkyltin alkoxides include butyltin trimethoxide, butyltin triethoxide, dibutyltin dimethoxide, dibutyltin diethoxide, dibutyltin diisoproxide, dibutyltin t-butoxide, dioctyltin dimethoxide, dioctyltin diethoxide, tributyltin methoxide, tributyltin ethoxide, triethyltin methoxide, trimethyltin methoxide and trioctyltin ethoxide. In addition, dialkyltin oxides may be used in the present invention. The dialkyltin oxide catalysts are described in U.S. Patent 4,206,143 which is incorporated by reference in its entirety. Examples of suitable dialkyltin oxides include those catalysts where the alkyl group contains from about 1 to 12 carbon atoms such as dimethyltin oxide, diethyltin oxide, dipropyltin oxide, dibutyltin oxide, dipentyltin oxide, dihexyltin oxide and the like. Dialkyltin oxides in which each alkyl group has from about 4 to 8 carbon atoms are preferred. The tin catalyst which is particularly preferred for use in the present invention is dibutyltin oxide.

Another class of catalysts useful in the method of this invention are metal alkoxides. The metal must have a valence of two to five. Examples of the metal alkoxides which may be suitable in the process of this invention may be represented by the formula:

$$M(OR^{15})_x$$

where M is a metal atom selected from the group consisting of lanthanum, niobium, tantalum, copper, zinc, tin, lead, antimony and bismuth. $R^{15}$ is a lower alkyl group of one to four carbon atoms and x is two to five depending on the valence of the metal atom. In addition to the metals noted above, metals in the same Periodic Table Groups may be expected to form active metal alkoxide compounds. The elements would be those metals of Groups IIIB, VB, IB, IIB, IVA and VA such as vanadium, silver, cadmium, germanium and the like.

The alkoxy groups contain alkyl substituents having one to four carbon atoms and can either be straight or branched. This definition includes methyl, ethyl, propyl, isopropyl, n-butyl, sec-butyl and isobutyl groups. Note that the catalyst contains no alkyl groups attached directly to the metal. An oxygen atom always intervenes.

Representative catalysts of this group include stannous dimethoxide, stannous tetramethoxide, lead diethoxide, zinc dimethoxide, copper dimethoxide, antimony trimethoxide, bismuth trimethoxide, bismuth tributoxide, tantalum ethoxide, niobium ethoxide and lanthanum diisopropoxide, among others.

The number of alkoxy groups should be either two through five, enough to satisfy the valence requirement of the metal atom.

Generally, the catalysts may be made by reacting a metal halide compound with an alkaline substance such as an amine or ammonia or alkali metal alkoxide in the presence of an alcohol (Example I, V, VI and VII). Alternatively, an alcohol and a metal oxide can be reacted together. Or an alkyl metal compound could be reacted with an alcohol. In reality, these catalysts are often not pure metal alkoxides. They may contain chlorine or other halogen atoms where an alkoxy group would be (see the catalyst preparation examples). The catalyst must have at least one alkoxy group. It could have up to four halogen substituents, depending on the valence of the metal. But as many substituents as possible should be alkoxy.

One of the advantages of the present invention is the ability to recycle catalyst residues. As one skilled in the art is aware, once the oxide containing catalyst has been reacted, the catalyst residue contains a different complex or complexes than originally charged to the virgin reaction. When a tin-catalyst is used, the complex is believed to contain Sn-N bonds generated from the reaction of organotin oxide and amine. These complexes are known as aminostannanes. Assuming the catalyst residue is still active, use of these catalyst residues are contemplated for use in the present invention as the sole catalyst or partial substitute for the above described catalysts.

The catalyst is present in an amount sufficient to catalyze the reaction of an acrylic acid ester or methacrylic acid ester with the amine to provide an N-substituted acrylamide or methacrylamide. The amount of catalyst will vary depending on the particular catalyst selected. Generally, the catalyst may be used in an amount of from about 0.05% to 20% and preferably from 0.1% to about 15%, based on the weight of the amines.

As mentioned above, an inhibiting amount of the nitroxyl containing inhibitor is present during the aminolysis reaction between the amine and the acrylic or methacrylic acid ester. The inhibitor is typically present in an amount ranging from about 10 ppm to 1500 ppm, and preferably from about 20 ppm to 600 ppm based on the weight of the ester.

The aminolysis reaction may be carried out in the absence of a solvent, in addition to the reactants, or in the presence of an additional suitable solvent such as benzene, toluene, xylene, mesitylene, or decahydronaphthalene. Preferably, the aminolysis reaction is carried out in the absence of a solvent.

Upon completion of the aminolysis reaction, the desired product may be in suspension. The solid product is isolated, e.g., filtration, from the remaining filtrate. The filtrate containing the catalyst residues and little if any by-product or polymer build-up may be used for subsequent preparation of additional product. To the filtrate can be added additional make-up amine and ester. If needed, make-up catalyst may be added.

The present invention will be further illustrated by the following examples which are not intended to limit the scope of the present invention.

Examples 1-16

The following examples were conducted to demonstrate the benefit of the present invention (Examples 2-5) versus other compounds (6-16) in a tin-catalyzed aminolysis reaction with (meth)acrylic esters. 184 grams (1.0 mole) of p-aminodiphenylamine (p-ADPA), 426 grams (4.26 moles) of methyl methacrylate (stabilized with 10 ppm of MEHQ), 12.4 grams (0.05 mole) dibutyltin oxide catalyst and inhibitor (the ppm based on the amount of methyl methacrylate) were charged into a 1 liter, 3 neck, round bottomed flask equipped with a mechanical teflon paddle stirrer, pot thermometer, nitrogen inlet, septum sampling port and a 1-foot silvered vacuum jacketed column packed with stainless steel protrusion packing topped with a solenoid activated distillation splitter. The system was brought to reflux under a slow nitrogen purge with the splitter set at a 1/1 reflux ratio (3 seconds on/3 seconds off) for the first 6 hours and a 1/2 ratio (3 seconds on/6 seconds off) for the last 5 hours of the reaction. A standard reaction time of 11 hours was followed in all cases. After the reaction time was completed, the column and splitter were removed and replaced with a short path distillation head and a 250 ml addition funnel. The remaining methyl methacrylate was distilled out under a nitrogen atmosphere while 250 ml of xylene was being concurrently added. When the pot temperature reached 130°C, an additional 200 ml of xylene was added to the pot all at once. The hot solution was then poured into a 1-liter beaker in an ice water bath. The product, N-(4-anilinophenyl) methacrylamide, was crystallized with stirring and allowed to stand in the ice bath until reaching 25-30°C. At this point, the product was filtered off and the filter cake washed portionwise with approximately 800 ml of xylene until the filtrate was almost colorless. The product was then dried over night in a 50-60°C circulating air oven. After drying, the crude weight was obtained and a crude yield calculated. 110 grams of the crude product was then dissolved in a 330 ml of methanol at 35°C with stirring. Filtration of this solution leaves the polymeric by-product as a dark semi-solid residue. Table I below lists the various inhibitors which were used, the concentration of the inhibitor, percent crude product yield and the weight percent of undesired polymer in the crude product. Example 1 was conducted in the presence of 10 ppm MEHQ as the control. Examples 2-5 represent the use of an inhibitor of the present invention added in addition to the 10 ppm MEHQ originally present in the methyl methacrylate. Examples 6-16 represent a number of prior art inhibitors that are known to those skilled in the art used in addition to 10 ppm MEHQ present in the methyl methacrylate.

TABLE I

| Example | Inhibitor | Inhibitor Concentration* | % Crude Yield | Weight % Polymer |
|---|---|---|---|---|
| 1 | Control | - | 94 | 4.27 |
| 2 | 2,2,6,6-tetramethyl-4-piperidinol-1-oxyl | 500 | 92 | 0.0 |
| 3 | " (duplicate) | 500 | 93 | 0.0 |
| 4 | 2,2,6,6-tetramethyl-4-piperidine-1-oxyl | 20 | 88 | Trace |
| 5 | Bis(2,2,6,6-tetramethyl-4-piperidinyl-N-oxyl) sebacate | 20 | 85 | Trace |
| 6 | 2,6-diphenylbenzoquinone | 500 | 96 | 8.36 |
| 7 | p-hydroxydiphenylamine | 500 | 94 | 3.36 |
| 8 | N,N'-diphenyl-p-phenylene diamine | 500 | 94 | 6.82 |
| 9 | Nitrosophenylhydroxylamine | 500 | 104 | 23.0 |
| 10 | N,N-dimethyl aniline | 500 | 92 | 3.36 |
| 11 | 4-nitrosophenol | 500 | 92 | 2.27 |
| 12 | α,N-diphenyl nitrone | 500 | 93 | 2.27 |
| 13 | thiocarbanilide | 500 | 94 | 3.90 |
| 14 | 2-(2-hydroxy-3-t-butyl-5-methyl-benzyl)-4-methyl-6-t-butylphenyl methacrylate | 500 | 93 | 3.45 |
| 15 | Diethylhydroxylamine | 500 | 93 | 3.60 |
| 16 | 4,6-dinitro-o-cresol | 20 | 88 | 2.27 |

* All inhibitor levels were relative to methyl methacrylate and in addition to 10 ppm MEHQ contained in the bulk methyl methacrylate.

As can be seen in Table I, use of a polymerization inhibitor containing at least one nitroxyl group resulted in trace, if any, amounts of polymer formed in the product, N-(4-anilinophenyl)methacrylamide. In comparison, with the control (Example 1) or with an inhibitor that did not contain a hindered nitroxyl group, substantial amounts of undesired polymer were found.

Example 17

The following example was conducted in order to demonstrate the advantage of the present invention by avoiding polymer build-up permitting recycle of the catalyst residue for further processing of product.

First Cycle Preparation of N-(4-anilinophenyl)methacrylamide

300 grams (1.19 moles) of pure N-(4-anilinophenyl)methacrylamide, 950 grams (9.5 moles) of methyl methacrylate (MMA), 184 grams (1.0 mole) of p-aminodiphenylamine, 24.8 grams (0.10 mole) of dibutyltin oxide and 0.475 grams (0.00275 moles) 2,2,6,6-tetramethyl-4-piperidinol-1-oxyl (4-H-TEMPO), free radical (500 ppm based on MMA) were charged into a 2 liter 3-neck flask equipped with a mechanical teflon paddle stirrer, thermometer, nitrogen inlet, septum sampling port and short Vigreux distillation set-up. The mixture was stirred at room temperature under slow nitrogen purge to dissolve N-(4-anilinophenyl) methacrylamide and p-aminodiphenylamine prior to obtaining an initial sample. The mixture was then heated to the point where the distillate temperature coming over was 85-92°C. Samples of the reaction mixture were taken periodically and the reaction followed by thin layer chromatography (TLC) using ethyl acetate as the solvent and iodine vapor as the developer. The reaction was run a total of 13.5 hours, and 341 grams of distillate was removed. After cooling to room temperature, 159 grams of MMA was added back to the reaction slurry. At this point, it was believed the reaction mixture contained about 552 grams N-(4-anilinophenyl)methacrylamide (300 grams original charge + 252 grams prepared) and 700 grams of MMA (905 grams original charge - 100 grams reacted - 309 grams distilled + 159 grams MMA addback). Since the solubility of N-(4-anilinophenyl)methacrylamide in MMA at room temperature is about 30% by weight, about 300 grams of the N-(4-anilinophenyl)methacrylamide would be dissolved in 700 grams of MMA leaving the 252 grams (1 mole) of generated N-(4-anilinophenyl)methacrylamide as a slurry suspended in the saturated solution.

The slurry was filtered to isolate N-(4-anilinophenyl)methacrylamide as fine crystalline needles. The dark brown filtrate was removed and the product cake washed carefully with 800 ml of xylene portionwise until the filtrate was clear. The product, N-(4-anilinophenyl)methacrylamide was green-yellow in color. The filtercake was dried in a circulating air oven at 60°C overnight to obtain 302 grams of product. The xylene filtrate (containing MMA, N-(4-anilinophenyl)methacrylamide and catalyst residues) was stripped of xylene under reduced pressure. The residue was then dissolved in 250 grams MMA and added back to the original filtrate. The filtrate solution concentrations now approximate the starting concentrations, that is, 950 grams MMA containing about 300 grams N-(4-anilinophenyl)-methacrylamide and 0.10 mole tin catalyst residues.

Second Cycle Preparation of N-(4-anilinophenyl) methacrylamide

To the modified filtrate from the first cycle was added an additional 184 grams (1.0 mole) of p-aminodiphenylamine in a 2 liter 3-neck flask equipped with a mechanical stirrer, nitrogen inlet, septum sampling port, port thermometer, one foot jacketed stainless steel protrusion packed column topped with a solenoid activated distillation splitter. The system was brought to reflux under a slow nitrogen purge with the splitter set at a 1/1 reflux ratio. The rheostat was adjusted to about 65 setting to maintain a distillate takeoff temperature of 85-92°C. Samples were taken via syringe periodically for TLC analysis. After 14 hours, the TLC spot intensities were about the same as those in the initial run after 13.5 hours. A total of 223 grams of distillate was obtained (about 32 grams methanol + 191 grams MMA). At this point, sufficient MMA was added back to the pot to bring the total to approximately 700 grams (950 grams start - 100 grams reacted - 191 distillate + 41 grams addback). The reaction slurry was then filtered at room temperature and worked up in an identical manner to the previous run. 245 grams of green-yellow product, N-(4-anilinophenyl)methacrylamide, were obtained after drying. The product appears identical to the product of the first cycle. Melting points of both products were 106-107.5°C indicating exceptionally high purity.

Examples 18-32

The following examples were conducted to demonstrate the effect of the presence or absence of a polymerization inhibitor containing a nitroxyl group. The inhibitor that was used was 2,2,6,6-tetramethyl-4-piperidinol-1-oxyl (4-H-TEMPO). The methyl methacrylate (MMA, stabilized with 10 ppm MEHQ), p-amino diphenylamine (p-ADPA), dibutyltin oxide (DBTO) and inhibitor, if any, were charged into a 3 neck, round bottom flask equipped with a mechanical teflon paddle stirrer, pot thermometer, nitrogen inlet, septum sampling port and a 1 foot silvered vacuum jacketed column packed with stainless steel protrusion packing topped with a solenoid activated distillation splitter. The system was brought to reflux under a slow nitrogen purge with the reflux ratio controlled to maintain an overhead temperature between 70°C and 90°C. Samples of the reaction mixture were periodically analyzed by gas chromatography using tetrahydrofuran as the solvent. A reaction time of between 10 and 15 hours was followed. After the reaction time was completed, the column and splitter were removed and replaced with a short path distillation head and a 250 ml addition funnel. The remaining MMA was distilled out using a nitrogen atmosphere. In some cases, 250 ml of xylene was concurrently added. When the pot temperature reached 130°C, 200 ml of additional xylene or MMA was added to the pot all at once. The hot solution was then poured into a glass beaker in an ice water bath. The product, N-(4-anilinophenyl)-methacrylamide, was crystallized with stirring and allowed to stand in the ice bath until reaching 25-30°C. At this point, the product was filtered off and the filter cake washed portionwise with xylene or MMA until the filtrate was almost colorless. The product was dried in a 50-60°C circulating air oven. After drying, the weight and purity of the desired product was ascertained, the percent yield of desired product calculated, and the percent polymer in the finished product was established by GPC. Table II below lists for each example the amounts of reactants used, the reaction solvent, whether a xylene chase was used, solvent used for the crystallization of the product, and the percentage of undesired polymer in the finished product.

TABLE II

| Example No. | 18 | 19 | 20 | 21 | 22 |
|---|---|---|---|---|---|
| MMA (g) | 404 | 607 | 607 | 428 | 338 |
| p-ADPA (g) | 184 | 184 | 184 | 178 | 184 |
| 4-H-TEMPO (g) | 0.20 | 0.31 | 0.50 | 0.50 | 0.50 |
| ppm | 495 | 511 | 824 | 1168 | 1480 |
| DBTO (g) | 12.4 | 24.8 | 24.8 | 10.0 | 12.4 |
| Reaction solvent | MMA | MMA | MMA | MMA/Xylene | MMA/Xylene |
| Xylene chase | Yes | None | None | None | Yes |
| Crystal. from | Xylene | MMA | MMA | MMA/Xylene | Xylene |
| Product (g) | 186 | 108 | 153 | 151 | 203 |
| % purity | 98.2 | 95.9 | 95.1 | 95.7 | 95.6 |
| % yield | 74 | 43 | 61 | 59 | 81 |
| Percent polymer in product | 0 | 0 | 0 | 0 | 0 |

TABLE II (Cont.)

| Example No. | 23 | 24 | 25 | 26 | 27 |
|---|---|---|---|---|---|
| MMA (g) | 619 | 505 | 505 | 404 | 2122 |
| p-ADPA (g) | 184 | 184 | 184 | 184 | 920 |
| 4-H-TEMPO (g) | 0.006 | - | - | - | - |
| ppm | 10 | - | - | - | - |
| DBTO (g) | 12.4 | 12.4 | 12.4 | 12.4 | 62.0 |
| Reaction solvent | MMA | MMA | MMA | MMA | MMA |
| Xylene chase | Yes | Yes | Yes | Yes | Yes |
| Crystal. from | Xylene | Xylene | Xylene | Xylene | Xylene |
| Product (g) | 211 | 233 | 242 | 230 | 1154 |
| % purity | 90.6 | 91.5 | 86.9 | 86.8 | 88.3 |
| % yield | 84 | 92 | 96 | 91 | 92 |
| Percent polymer in product | 0 | 2.3 | 6.1 | 4.7 | 4.8 |

TABLE II (Cont.)

| Example No. | 28 | 29 | 30 | 31 | 32 |
|---|---|---|---|---|---|
| MMA (g) | 2122 | 505 | 505 | 505 | 505 |
| p-ADPA (g) | 920 | 184 | 184 | 184 | 184 |
| 4-H-TEMPO (g) | - | - | - | - | - |
| ppm | - | - | - | - | - |
| DBTO (g) | 62.0 | 12.4 | 12.4 | 12.4 | 12.4 |
| Reaction solvent | MMA | MMA | MMA | MMA | MMA |
| Xylene chase | Yes | Yes | Yes | Yes | Yes |
| Crystal. from | Xylene | Xylene | Xylene | Xylene | Xylene |
| Product (g) | 1007 | 252 | 246 | 236 | 231 |
| % purity | 94.0 | 87.0 | 94.3 | 88.6 | N/A |
| % yield | 80 | 100 | 98 | 94 | 92 |
| Percent polymer in product | 0.7 | 7.5 | 4.4 | 2.2 | 7.0 |

As can be seen from Examples 18-23, the presence of an inhibitor containing at least one nitroxyl group results in a product of high purity and a significant avoidance of thermal polymerization. Examples 24-32 demonstrate that whereas one may produce products in relatively high yields, there were significant amounts of polymer product.

Examples 33-42

The following examples were conducted to study the effect of inhibitor levels in a tin-catalyzed aminolysis reaction. The polymerization inhibitor was 2,2,6,6-tetramethyl-4-piperidinol-1-oxyl (4-H-TEMPO). The methyl

methacrylate (MMA, stabilized with 10 ppm MEHQ), p-amino diphenylamine (p-ADPA), dibutyltin oxide (DBTO) and inhibitor, if any, were charged into a 3 neck, round bottom flask equipped with a mechanical teflon paddle stirrer, pot thermometer, nitrogen inlet, septum sampling port and a 1 foot silvered vacuum jacketed column packed with stainless steel protrusion packing topped with a solenoid activated distillation splitter. The system was brought to reflux under a slow nitrogen purge with the reflux ratio controlled to maintain an overhead temperature between 70°C and 90°C. Samples of the reaction mixture were periodically analyzed by gas chromatography using tetrahydrofuran as the solvent. A reaction time of between 10 to 15 hours was followed. After the reaction time was completed, the column and splitter were removed and replaced with a short path distillation head and a 250 ml addition funnel. The remaining MMA was distilled out rising a nitrogen atmosphere. When the pot temperature reached 130°C, 200 ml of xylene or MMA was added to the pot all at once. The hot solution was then poured into a glass beaker in an ice bath. The product, N-(4-anilinophenyl)methacrylamide, was crystallized with stirring and allowed to stand in the ice bath until reaching 25-30°C. At this point, the product was filtered off and the filter cake washed portionwise with xylene or MMA until the filtrate was almost colorless. The product was dried in a 50-60°C circulating air oven. After drying, the weight and purity of desired product was ascertained, the percent yield of desired product calculated and the percent polymer in the finished product was established by GPC. Table III below lists for each example the amounts of reactants used, the amount of 4-H-TEMPO in grams and ppm based on MMA, the reaction solvent, whether a xylene chase was used, the solvent used for the crystallization of the product, the percentage of undesired polymer in the finished product, the weight, purity and the percent yield of the desired product.

TABLE III

| Example No. | 33 | 34 | 35 | 36 | 37 |
| --- | --- | --- | --- | --- | --- |
| MMA (g) | 607 | 607 | 619 | 607 | 609 |
| p-ADPA (g) | 184 | 184 | 184 | 184 | 184 |
| 4-H-TEMPO (g) | 0 | 0 | 0.006 | 0.006 | 0.015 |
| ppm | 0 | 0 | 10 | 10 | 25 |
| DBTO (g) | 12.4 | 12.4 | 12.4 | 12.4 | 12.4 |
| Reaction solvent | MMA | MMA | MMA | MMA | MMA |
| Xylene chase | None | Yes | Yes | None | None |
| Crystal. from | MMA | Xylene | Xylene | MMA | MMA |
| Product (g) | 135 | 240 | 211 | 187 | 137 |
| % purity | 94.7 | 83.3 | 90.6 | 93.8 | 96.8 |
| % yield | 54 | 95 | 84 | 74 | 54 |
| Percent polymer in product | 0.4 | 3.7 | 0 | 0 | 0 |

TABLE III (Cont.)

| Example No. | 38 | 39 | 40 | 41 | 42 |
|---|---|---|---|---|---|
| MMA (g) | 615 | 607 | 404 | 607 | 607 |
| p-ADPA (g) | 184 | 184 | 184 | 184 | 184 |
| 4-H-TEMPO (g) | 0.05 | 0.10 | 0.20 | 0.31 | 0.50 |
| ppm | 81 | 165 | 495 | 511 | 824 |
| DBTO (g) | 12.4 | 12.4 | 12.4 | 24.8 | 24.8 |
| Reaction solvent | MMA | MMA | MMA | MMA | MMA |
| Xylene chase | None | None | Yes | None | None |
| Crystal. from | MMA | MMA | Xylene | MMA | MMA |
| Product (g) | 158 | 145 | 186 | 108 | 153 |
| % purity | 99.6 | 98.7 | 98.2 | 95.9 | 95.1 |
| % yield | 63 | 58 | 74 | 43 | 61 |
| Percent polymer in product | 0 | 0 | 0 | 0 | 0 |

The data listed in Table III above would indicate that relatively small amounts, i.e., 10 ppm are effective in the avoidance of detectable polymer formation. In addition, the data indicate that as the amount of inhibitor is increased, there was no significant effect on product purity.

Example 43

The following examples were conducted to demonstrate the effect of recycling tin-catalyst residues in a series of aminolysis reactions. The general procedure of Examples 33-43 was repeated except the tin-catalyst containing filtrate was recycled and make-up reactants were added. In all runs, MMA was the reaction solvent, there was no xylene chase and the product was crystallized from MMA. The filtrate was recycled eleven times.

After the original run and each recycle, the amount of undesired polymer and the weight, purity and yield of product was ascertained. This data appears below in Table IV as well as the weight of the recycled filtrate, the amount of 4-H-TEMPO, the amount of DBTO, MMA and p-ADPA.

TABLE IV

| Run | Original | 1st Recycle | 2nd Recycle | 3rd Recycle | 4th Recycle | 5th Recycle |
|---|---|---|---|---|---|---|
| MMA | 607 | 546 | 447 | 410 | 309 | 427 |
| p-ADPA | 184 | 177 | 162 | 161 | 183 | 178 |
| 4-H-TEMPO (g) | 0.50 | 0.50 | 0.50 | 0.50 | 0.50 | 0.50 |
| ppm | 824 | 849 | 911 | 883 | 965 | 917 |
| DBTO (g) | 24.8 | 12.4 | 7.5 | 11.1 | 10.0 | 10.0 |
| Recycle filtrate (g) | 0 | 80 | 200 | 233 | 313 | 196 |
| Product (g) | 153 | 110 | 163 | 173 | 178 | 173 |
| % Purity | 95.1 | 96.6 | 95.1 | 95.1 | 97.1 | 99.4 |
| % Yield | 61 | 39 | 47 | 59 | 53 | 54 |
| Percent polymer in product | 0 | 0 | 0 | 0 | 0 | 0 |

TABLE IV (Cont.)

| Run | 6th Recycle | 7th Recycle | 8th Recycle | 9th Recycle | 10th Recycle | 11th Recycle |
|---|---|---|---|---|---|---|
| MMA | 524 [1] | 420 | 420 | 603 [2] | 481 [3] | 513 [4] |
| p-ADPA | 177 | 184 | 184 | 184 | 144 | 147 |
| 4-H-TEMPO (g) | 0.50 | 0.50 | 0.50 | 0.50 | 0.50 | 0.50 |
| ppm | 753 | 1035 | 949 | 729 | 731 | 699 |
| DBTO (g) | 8.3 | 5.0 | 5.0 | 5.3 | 5.0 | 10.0 |
| Recycle filtrate (g) | 223 | 103 | 193 | 160 | 312 | 190 |
| Product (g) | 193 | 122 | 170 | 160 | 113 | 174 |
| % Purity | 93.9 | 85.3 | 98.0 | 96.9 | 98.4 | 99.6 |
| % Yield | 62 | 41 | 47 | 46 | 26 | 59 |
| Percent polymer in product | 0 | 0 | 0 | 0 | 0 | 0 |

[1] Make-up MMA charged: 407 g initially + 117 g for crystallization

[2] Make-up MMA charged: 430 g initially + 173 g for crystallization

[3] Make-up MMA charged: 355 g initially + 126 g for crystallization

[4] Make-up MMA charged: 469 g initially + 44 g for crystallization

Example 43 demonstrates that use of a polymerization inhibitor containing at least one nitroxyl group allows the recycle of tin-containing catalyst residues. As can be seen in Table IV above, in all of the recycle reactions, the purity of the end product remained relatively high without any major reduction in the percentage of yield.

The above examples are given by way of illustration only and are not intended as limitations in the scope of this invention, as defined by the appended claims.

## Claims

1. A process for the catalyzed synthesis of N-substituted acrylamides characterized by reacting at a temperature ranging from 70° to 150°C an alkyl ester of acrylic or methacrylic acid with an aliphatic or aromatic amine in the presence of a polymerization inhibitor containing a nitroxyl group and in the presence of 0.05% to 20% based on the weight of the amine of a catalyst compound selected from the group consisting of an alkyltin oxide, trialkyltin alkoxide, a dialkyltin oxide, or a metal alkoxide having the formula:

$$M(OR^{15})_x$$

wherein M is a metal selected from the group consisting of lanthanum, niobium, tantalum, copper, zinc, tin, lead, antimony and bismuth, $R^{15}$ is a lower alkyl radical having from 1 to about 4 carbon atoms and x is two through five depending on the valence of the metal atom or residue of said catalyst compound wherein the polymerization inhibitor is selected from the group consisting of:

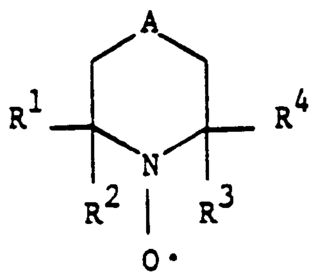
(I),

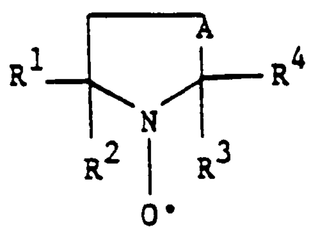
(II),

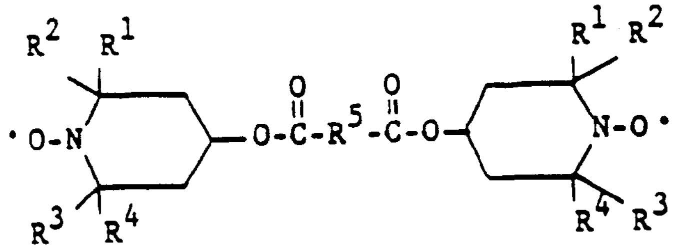
(III),

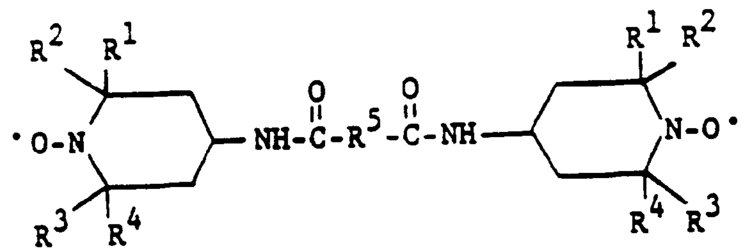
(IV),

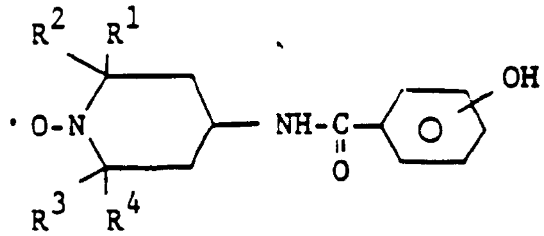

(V),

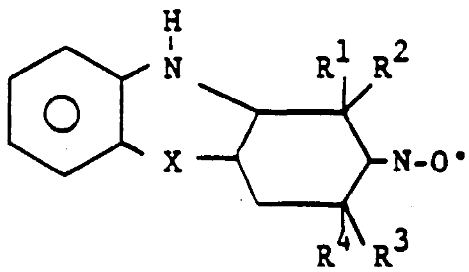

(VI),

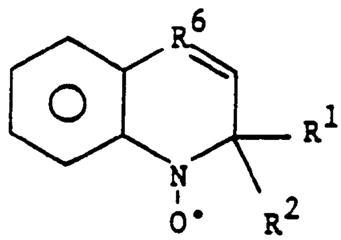

(VII),

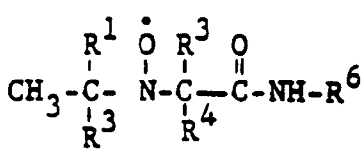

(VIII),

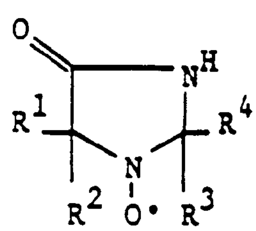

(IX),

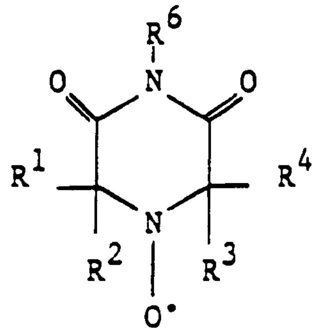

(X),

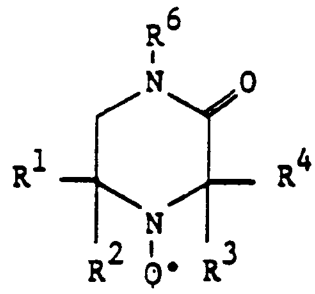

(XI),

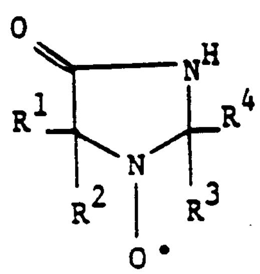

(XII),

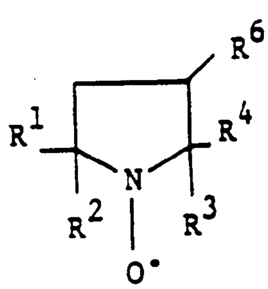

(XIII),

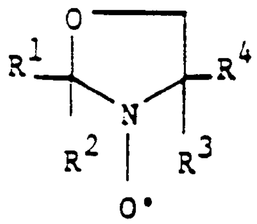 (XIV), and 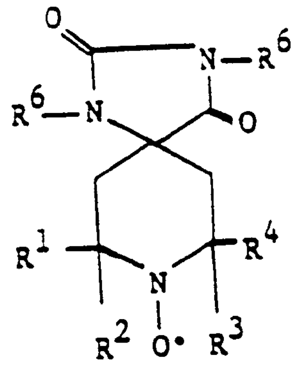 (XV)

wherein $R^1$, $R^2$, $R^3$, and $R^4$ are independently linear or non-alpha branched alkyl radicals having 1 to 9 carbon atoms or one or both of $R^1$ and $R^2$ or $R^3$ and $R^4$ form a cycloalkyl ring having 5 to 7 carbon atoms inclusive of the carbon atom in the hetero ring; wherein A is $-CH_2-$,

$$-\underset{|}{C}=O, \quad -\underset{|}{C}H-OR^8-,$$

$$-CH-O-\overset{O}{\overset{\|}{C}}-R^8 \quad ,$$

$$-\overset{|}{C}H-NHR^7, \quad -\overset{|}{C}H-NR^7R^8$$

wherein $R^7$ and $R^8$ are independently hydrogen radicals, alkyl radicals having 1 to 18 carbon atoms, aryl radicals having from 6 to 14 carbon atoms or aralkyl radicals having from 7 to 15 carbon atoms; wherein $R^5$ is an alkylene radical having from 5 to 10 carbon atoms, an arylene radical having from 6 to 14 carbon atoms or an aralkylene radical having from 7 to 15 carbon atoms; wherein $R^6$ is independently an alkyl radical having 1 to 18 carbon atoms, an aryl radical having 6 to 14 carbon atoms or an aralkyl radical and wherein X is NH, S or O.

2. The process of claim 1 characterized in that the polymerization inhibitor is selected from the group consisting of the formula II and III, wherein $R^1$, $R^2$, $R^3$, and $R^4$ are independently a linear alkyl radical having 1 carbon atom; $R^5$ is an alkylene radical having 8 carbon atoms or an arylene radical having 6 carbon atoms and A is $-CH_2-$ or $-CH-OH$.

3. The process of claim 1 characterized in that said acrylic or methacrylic acid ester is of the formula:

$$CH_2=\overset{R^9}{\overset{|}{C}}-\overset{O}{\overset{\|}{C}}-O-R^{10}$$

where $R^9$ is a hydrogen radical or methyl radical and $R^{10}$ is an alkyl radical having from about 1 to 4 carbon atoms.

4. The process of claim 1 characterized in that said amine is of the formula:

$$HNR^{11}R^{12}$$

wherein $R^{11}$ is a hydrogen radical or alkyl radical having from about 1 to 4 carbon atoms and $R^{12}$ is an alkyl radical having from about 1 to about 10 carbon atoms, an alkaryl radical having from about 7 to about 10 carbon atoms, an aralkyl radical having from about 7 to 10 carbon atoms, an alkoxyalkyl having from

about 2 to about 20 carbon atoms, or

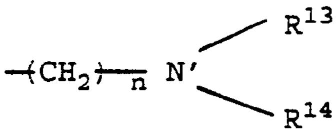

wherein n is an integer of from 2 to 6 and $R^{13}$ and $R^{14}$ are independently alkyl radicals having 1 to 4 carbon atoms or $R^{13}$ and $R^{14}$, when taken jointly, are combined with the N' atom to form a heterocyclic ring group selected from the group consisting of morpholine pyrrolidine and piperidine ring groups.

5. The process of claim : characterized in that the molar ratio of said amine to said alkyl ester of acrylic or methacrylic acid ranges from about 1:1 to about 1:100.

6. The process of claim 1 characterized in that said polymerization inhibitor is present in an amount ranging from about 10 ppm to about 1500 ppm based on the weight of the ester.

7. The process of claim 1 characterized in that said catalyst is a dialkyltin oxide wherein each alkyl radical is independently an alkyl radical having from about 1 to 12 carbon atoms.

8. The process of claim 1 characterized in that said dialkyltin oxide is dibutyltin oxide.

9. The process of claim 1 characterized in that said catalyst is an alkyltin alkoxide, dialkyltin alkoxide or trialkyltin alkoxide.

10. The process of claim 9 characterized in that said catalyst is selected from the group consisting of butyltin trimethoxide, butyltin triethoxide, dibutyltin dimethoxide, dibutyltin diethoxide, dibutyltin diisoproxide, dibutyltin t-butoxide , dioctyltin dimethoxide, dioctyltin diethoxide, tributyltin methoxide, tributyltin ethoxide, triethyltin methoxide, trimethyltin methoxide and trioctyltin ethoxide.

11. The process of claim 1 characterized in that said process is conducted in the presence of a solvent selected from the group consisting of toluene, xylene, mesitylene and decahydronaphthalene.

12. The process of claim 1 characterized by:
(a) reacting at a temperature ranging from 70°C to 150°C an alkyl ester of acrylic or methacrylic acid ester of the formula:

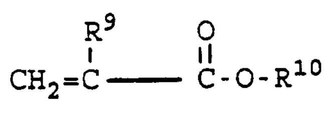

with an amine of the formula:

$HNR^{11}R^{12}$

in the presence of a polymerization inhibitor selected from the group consisting of:

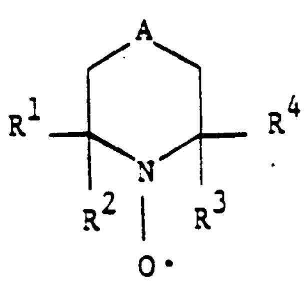

(I),

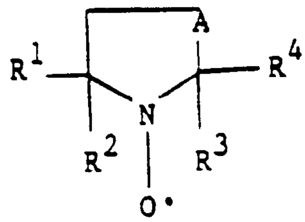

(II),

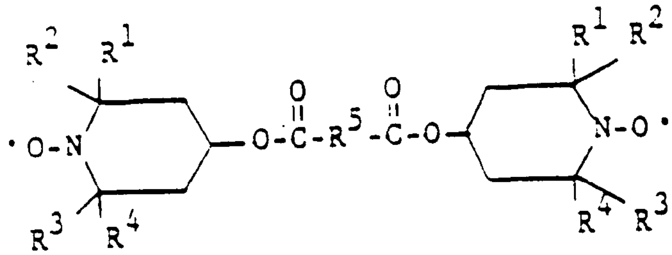

(III),

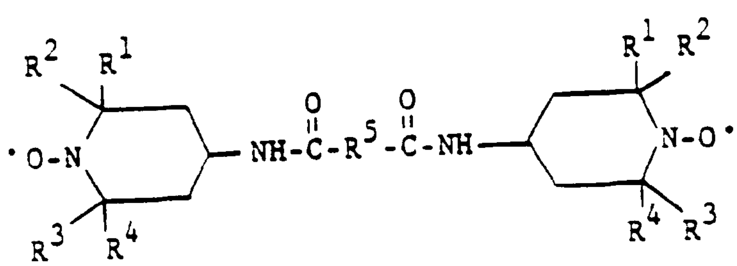

(IV),

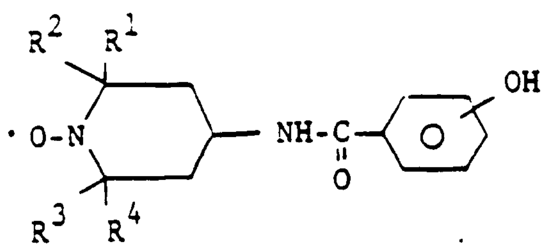

(V),

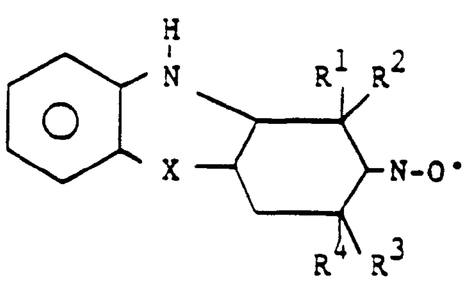

(VI),

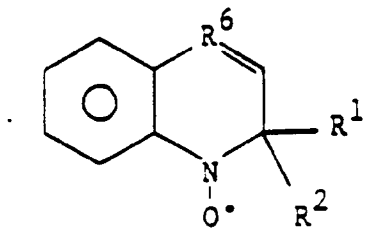

(VII),

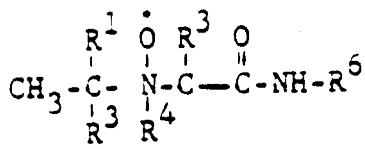

(VIII),

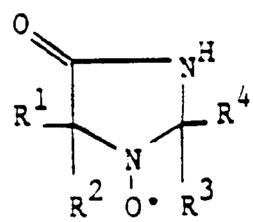

(IX),

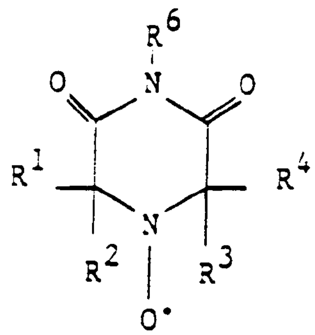

(X),

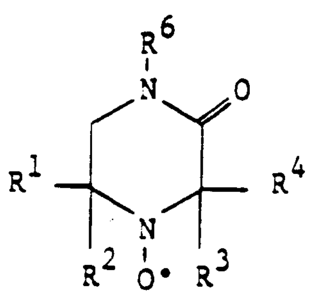

(XI),

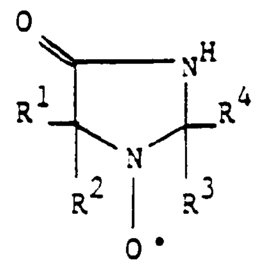

(XII),

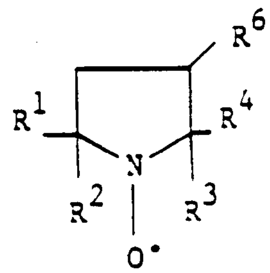

(XIII),

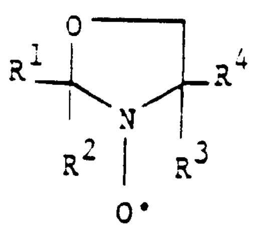

(XIV), and

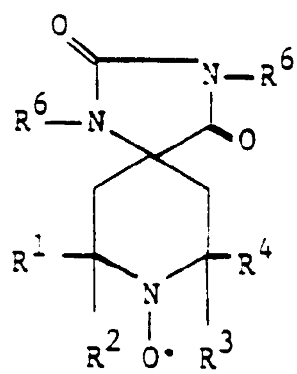

(XV)

in the presence of 0.05% to 20% based on the weight of the amine of a catalyst compound selected from the group consisting of an alkyltin alkoxide, trialkyl alkoxide, a dialkyltin oxide or a metal alkoxide having the formula:

$$M(OR^{15})_x$$

wherein $R^1$, $R^2$, $R^3$, and $R^4$ are independently linear or non-alpha branched alkyl radicals having 1 to 9 carbon atoms or one or both of $R^1$ and $R^2$ or $R^3$ and $R^4$ form a cycloalkyl ring having 5 to 7 carbon atoms inclusive of the carbon atom in the hetero ring; wherein A is $-CH_2-$,

-C=O, -CH-OR⁸-,

-CH-O-C(=O)-R⁸ ,

-CH-NHR⁷, -CH-NR⁷R⁸

wherein $R^7$ and $R^8$ are independently hydrogen radicals, alkyl radicals having 1 to 18 carbon atoms,

aryl radicals having from 6 to 14 carbon atoms or aralkyl radicals having from 7 to 15 carbon atoms; wherein $R^5$ is an alkylene radical having from 5 to 10 carbon atoms, an arylene radical having from 6 to 14 carbon atoms or an aralkylene radical having from 7 to 15 carbon atoms; wherein $R^6$ is independently an alkyl radical having 1 to 18 carbon atoms, an aryl radical having 6 to 14 carbon atoms or an aralkyl radical and wherein X is NH, S or O; wherein $R^9$ is a hydrogen radical or methyl radical and $R^{10}$ is an alkyl radical having from about 1 to 4 carbon atoms; wherein $R^{11}$ is a hydrogen radical or alkyl radical having from about 1 to 4 carbon atoms and $R^{12}$ is an alkyl radical having from about 1 to about 20 carbon atoms, an alkaryl radical having from about 7 to about 20 carbon atoms, an aralkyl radical having from about 7 to 20 carbon atoms, an alkoxyalkyl having from about 2 to about 20 carbon atoms, or

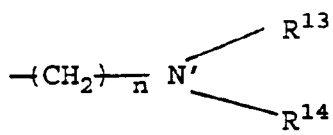

wherein n is an integer of from 2 to 6 and $R^{13}$ and $R^{14}$ are independently alkyl radicals having 1 to 4 carbon atoms or $R^{13}$ and $R^{14}$, when taken jointly, are combined with the N' atom to form a heterocyclic ring group selected from the group consisting of morpholine, pyrrolidine and piperidine ring groups; wherein M is a metal selected from the group consisting of lanthanum, niobium, tantalum, copper, zinc, tin, lead, antimony and bismuth, $R^{15}$ is a lower alkyl radical having from 1 to about 4 carbon atoms and x is two through five depending on the valence of the metal atom;

(b) isolating the N-substituted acrylamide from the filtrate containing the catalyst residue; and

(c) repeating step (a) with the catalyst residue as the catalyst compound.

**13.** The process of claim 12 wherein additional amounts of catalyst is added to the catalyst residue.

## Patentansprüche

**1.** Verfahren zur katalysierten Synthese von N-substituierten Acrylamiden, gekennzeichnet durch Umsetzen eines Alkylesters von Acryl- oder Methacrylsäure bei einer Temperatur im Bereich von 70 bis 150°C mit einem aliphatischen oder aromatischen Amin in Gegenwart eines Polymerisationsinhibitors, der eine Nitroxylgruppe enthält, und in Gegenwart von 0,05 bis 20%, bezogen auf das Gewicht des Amins, einer Katalysatorverbindung, die ausgewählt ist aus der aus einem Alkylzinnoxid, Trialkylzinnalkoxid, Dialkylzinnoxid oder Metallalkoxid der Formel

$$M(OR^{15})_x$$

bestehenden Gruppe, wobei M ein Metall ist, das ausgewählt ist aus der aus Lanthan, Niob, Tantal, Kupfer, Zink, Zinn, Blei, Antimon und Wismut bestehenden Gruppe, $R^{15}$ ein niederer Alkylrest mit 1 bis etwa 4 Kohlenstoffatomen ist und x 2 bis 5 ist, in Abhängigkeit von der Wertigkeit des Metallatoms oder des Restes der Katalysatorverbindung, worin der Polymerisationsinhibitor ausgewählt ist aus der aus:

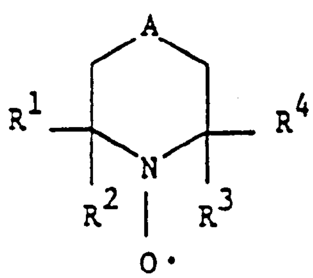

(I),

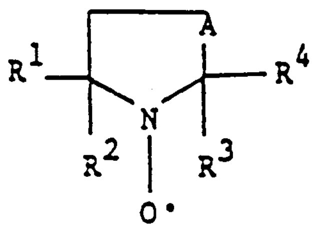

(II),

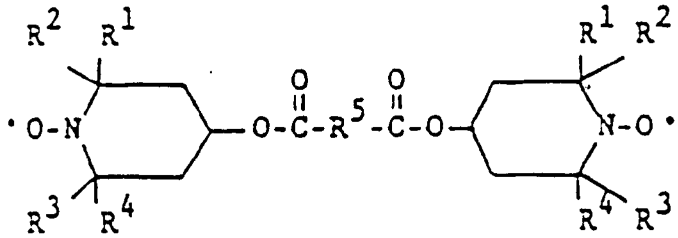

(III),

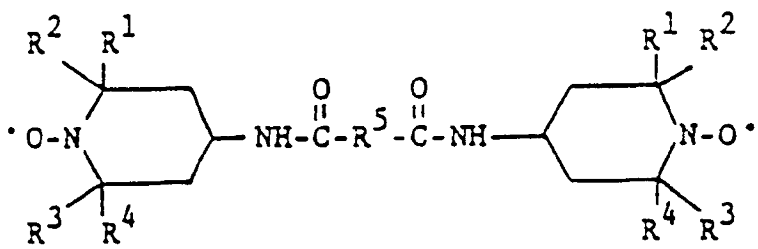

(IV),

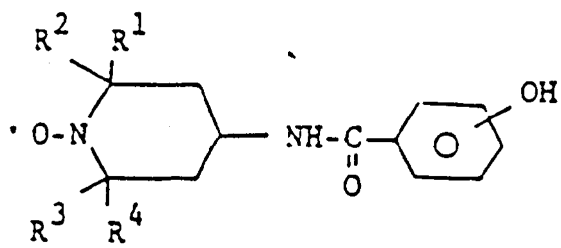

(V),

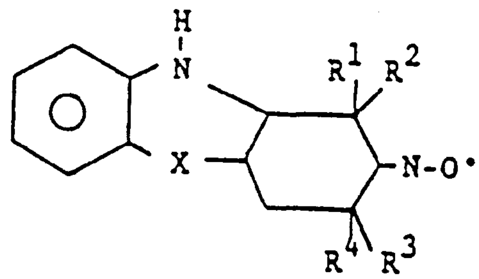

(VI),

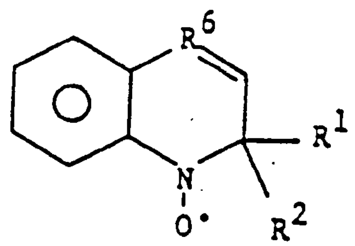

(VII),

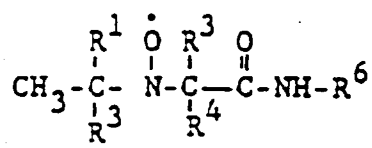

(VIII),

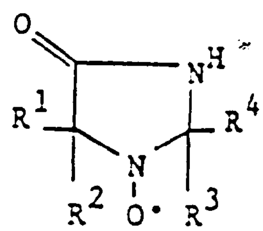

(IX),

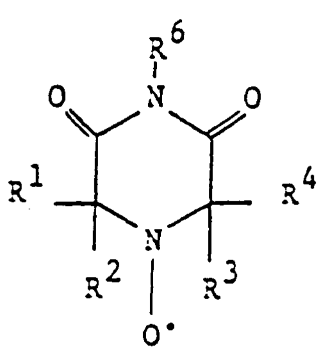

(X),

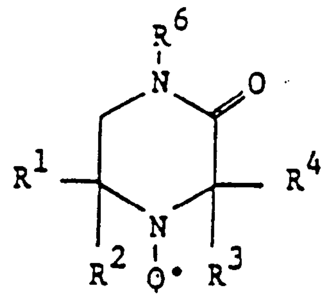

(XI),

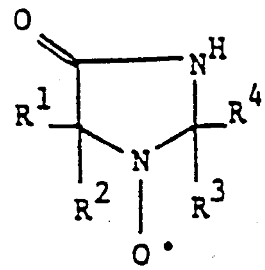

(XII),

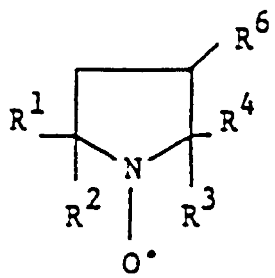

(XIII),

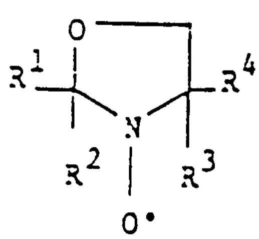

(XIV) und

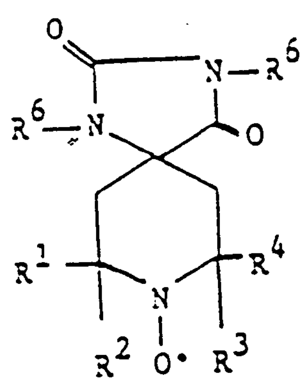

(XV)

bestehenden Gruppe, wobei $R^1$, $R^2$, $R^3$ und $R^4$ unabhängig lineare oder nicht-$\alpha$-verzweigte Alkylreste mit 1 bis 9 Kohlenstoffatomen sind oder einer oder beide Reste $R^1$ und $R^2$ oder $R^3$ und $R^4$ einen Cycloalkylring mit 5 bis 7 Kohlenstoffatomen, einschließlich des Kohlenstoffatoms in dem Heteroring, bilden; worin A

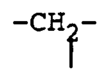, 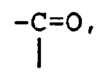,

-CH-OR$^8$

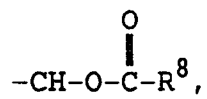, 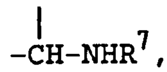, 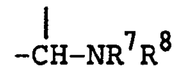

ist, worin $R^7$ und $R^8$ unabhängig Wasserstoffreste, Alkylreste mit 1 bis 18 Kohlenstoffatomen, Arylreste mit 6 bis 14 Kohlenstoffatomen oder Aralkylreste mit 7 bis 15 Kohlenstoffatomen sind; worin $R^5$ ein Alkylenrest mit 5 bis 10 Kohlenstoffatomen, ein Arylenrest mit 6 bis 14 Kohlenstoffatomen oder ein Aralkylenrest mit 7 bis 15 Kohlenstoffatomen ist; worin $R^6$ unabhängig ein Alkylrest mit 1 bis 18 Kohlenstoffatomen, ein Arylrest mit 6 bis 14 Kohlenstoffatomen oder ein Aralkylrest ist und worin X NH, S oder O ist.

2. Verfahren von Anspruch 1, dadurch gekennzeichnet, daß der Polymerisationsinhibitor ausgewählt ist aus der aus den Formeln II und III bestehenden Gruppe, worin $R^1$, $R^2$ $R^3$ und $R^4$ unabhängig ein linearer Alkylrest mit 1 Kohlenstoffatom sind; $R^5$ ein Alkylenrest mit 8 Kohlenstoffatomen oder ein Arylenrest mit 6 Kohlenstoffatomen ist und A $-CH_2-$ oder -CH-OH ist.

3. Verfahren nach Anspruch 1, dadurch gekennzeichnet, daß der Acryl- oder Methacrylsäureester die folgende Formel hat:

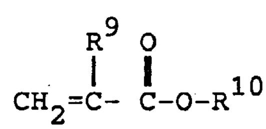

worin R9 ein Wasserstoffrest oder Methylrest ist und R10 ein Alkylrest mit etwa 1 bis 4 Kohlenstoffatomen ist.

4. Verfahren nach Anspruch 1, dadurch gekennzeichnet, daß das Amin die folgende Formel hat:

$$HNR^{11}R^{12}$$

worin $R^{11}$ ein Wasserstoffrest oder Alkylrest mit etwa 1 bis 4 Kohlenstoffatomen ist und $R^{12}$ ein Alkylrest mit etwa 1 bis etwa 20 Kohlenstoffatomen, ein Alkarylrest mit etwa 7 bis etwa 20 Kohlenstoffatomen, ein Aralkylrest mit etwa 7 bis 20 Kohlenstoffatomen, ein Alkoxyalkyl mit etwa 2 bis etwa 20 Kohlenstoffatomen oder:

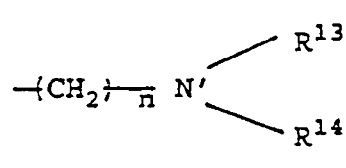

ist, worin n eine ganze Zahl von 2 bis 6 ist und $R^{13}$ und $R^{14}$ unabhängig Alkylreste mit 1 bis 4 Kohlenstoffatomen sind oder $R^{13}$ und $R^{14}$ zusammen mit dem N'-Atom eine heterocyclische Ringgruppe bilden, die ausgewählt ist aus der aus Morpholin-, Pyrrolidin- und Piperidinringgruppen bestehenden Gruppe.

5. Verfahren nach Anspruch 1, dadurch gekennzeichnet, daß das Molverhältnis von Amin zu Alkylester von Acryl- oder Methacrylsäure im Bereich von etwa 1:1 bis etwa 1:100 liegt.

6. Verfahren nach Anspruch 1, dadurch gekennzeichnet, daß der Polymerisationsinhibitor in einer Menge von etwa 10 ppm bis etwa 1500 ppm, bezogen auf das Gewicht des Esters, vorhanden ist.

7. Verfahren nach Anspruch 1, dadurch gekennzeichnet, daß der Katalysator ein Dialkylzinnoxid ist, worin jeder Alkylrest unabhängig ein Alkylrest mit etwa 1 bis 12 Kohlenstoffatomen ist.

8. Verfahren nach Anspruch 1, dadurch gekennzeichnet, daß das Dialkylzinnoxid Dibutylzinnoxid ist.

9. Verfahren nach Anspruch 1, dadurch gekennzeichnet, daß der Katalysator ein Alkylzinnalkoxid, Dialkylzinnalkoxid oder Trialkylzinnalkoxid ist.

10. Verfahren nach Anspruch 9, dadurch gekennzeichnet, daß der Katalysator ausgewählt ist aus der aus Butylzinntrimethoxid, Butylzinntriethoxid, Dibutylzinndimethoxid, Dibutylzinndiethoxid, Dibutylzinndiisopropoxid, Dibutylzinn-t-butoxid, Dioctylzinndimethoxid, Dioctylzinndiethoxid, Tributylzinnmethoxid, Tributylzinnethoxid, Triethylzinnmethoxid, Trimethylzinnmethoxid und Trioctylzinnethoxid bestehenden Gruppe.

11. Verfahren nach Anspruch 1, dadurch gekennzeichnet, daß das Verfahren in Gegenwart eines Lösungsmittels durchgeführt wird, das ausgewählt ist aus der aus Toluol, Xylol, Mesitylen und Decahydronaphthalin bestehenden Gruppe.

12. Verfahren nach Anspruch 1, gekennzeichnet durch:

(a) Umsetzen bei einer Temperatur im Bereich von 70°C bis 150°C eines Alkylesters von Acryl- oder Methacrylsäure der Formel:

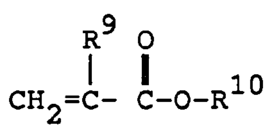

mit einem Amin der Formel:

$$HNR^{11}R^{12}$$

in Gegenwart eines Polymerisationsinhibitors, der ausgewählt ist aus der aus:

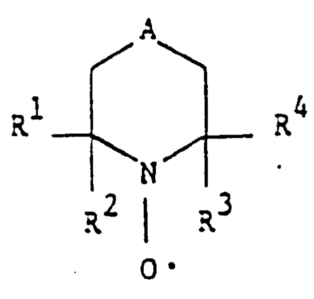

(I),

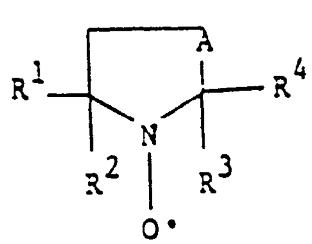

(II),

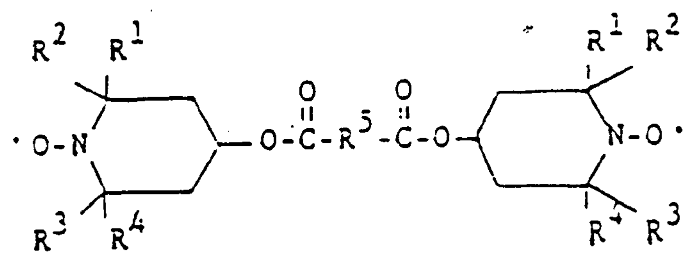

(III),

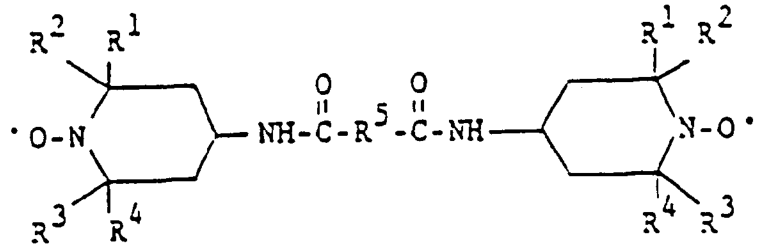

(IV),

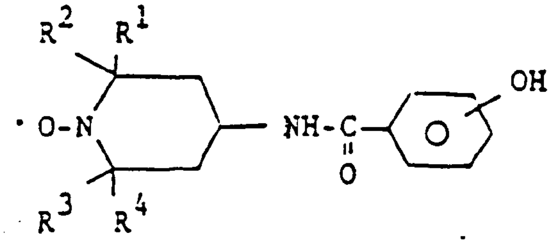

(V),

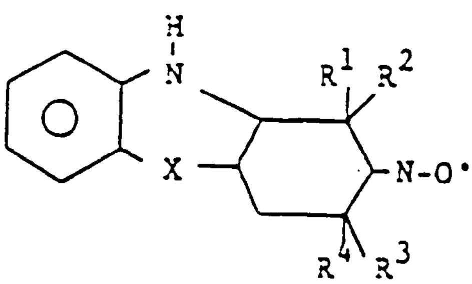

(VI),

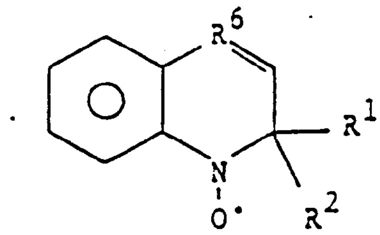

(VII),

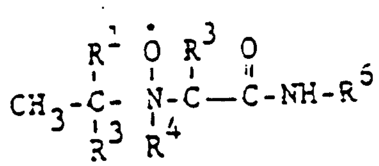

(VIII),

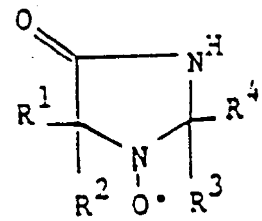

(IX),

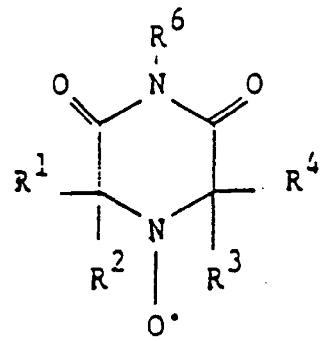

(X),

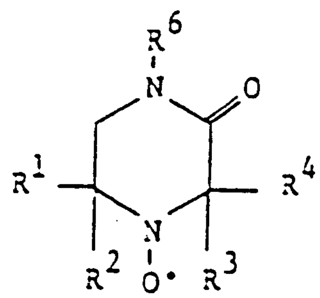

(XI),

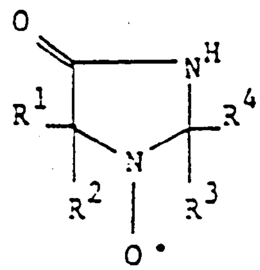

(XII),

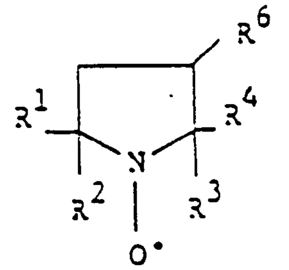

(XIII),

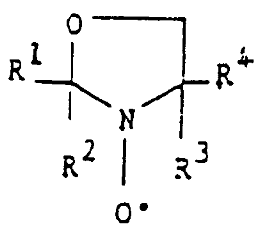

(XIV) und

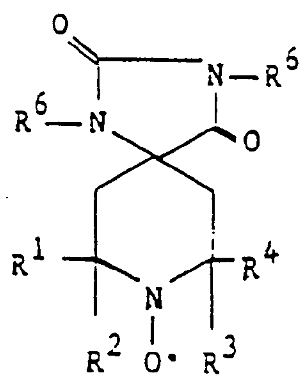

(XV)

bestehenden Gruppe in Gegenwart von 0,05% bis 20%, bezogen auf das Gewicht des Amins, einer Katalysatorverbindung, die ausgewählt ist aus der aus einem Alkylzinnalkoxid, Trialkylzinnalkoxid, Dialkylzinnoxid oder einem Metallalkoxid der Formel:

$$M(OR^{15})_x$$

bestehenden Gruppe, worin $R^1$, $R^2$, $R^3$ und $R^4$ unabhängig lineare oder nicht-α-verzweigte Alkylreste mit 1 bis 9 Kohlenstoffatomen sind oder einer oder beide Reste $R^1$ und $R^2$ oder $R^3$ und $R^4$ einen Cycloalkylring mit 5 bis 7 Kohlenstoffatomen, einschließlich des Kohlenstoffatoms in dem Heteroring, bilden; worin A $-CH_2-$,

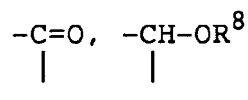

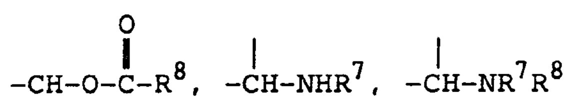

ist, worin $R^7$ und $R^8$ unabhängig Wasserstoffreste, Alkylreste mit 1 bis 18 Kohlenstoffatomen, Arylreste mit 6 bis 14 Kohlenstoffatomen oder Aralkylreste mit 7 bis 15 Kohlenstoffatomen sind; worin $R^5$ ein Alkylenrest mit 5 bis 10 Kohlenstoffatomen, ein Arylenrest mit 6 bis 14 Kohlenstoffatomen oder ein

Aralkylenrest mit 7 bis 15 Kohlenstoffatomen ist; worin $R^6$ unabhängig ein Alkylrest mit 1 bis 18 Kohlenstoffatomen, ein Arylrest mit 6 bis 14 Kohlenstoffatomen oder ein Aralkylrest ist und worin X NH, S oder O ist; worin $R^9$ ein Wasserstoffrest oder Methylrest ist und $R^{10}$ ein Alkylrest mit etwa 1 bis 4 Kohlenstoffatomen ist; worin $R^{11}$ ein Wasserstoffrest oder Alkylrest mit etwa 1 bis 4 Kohlenstoffatomen ist und $R^{12}$ ein Alkylrest mit etwa 1 bis etwa 20 Kohlenstoffatomen, ein Alkarylrest mit etwa 7 bis etwa 20 Kohlenstoffatomen, ein Aralkylrest mit etwa 7 bis 20 Kohlenstoffatomen, ein Alkoxyalkyl mit etwa 2 bis etwa 20 Kohlenstoffatomen oder

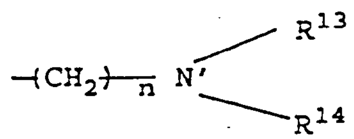

ist, worin n eine ganze Zahl von 2 bis 6 ist und $R^{13}$ und $R^{14}$ unabhängig Alkylreste mit 1 bis 4 Kohlenstoffatomen sind oder $R^{13}$ und $R^{14}$ zusammen in Kombination mit dem N'-Atom eine heterocyclische Ringgruppe bilden, die ausgewählt ist aus der aus Morpholin-, Pyrrolidin- und Piperidinringgruppen bestehenden Gruppe; worin M ein Metall ist, das ausgewählt ist aus der aus Lanthan, Niob, Tantal, Kupfer, Zink, Zinn, Blei, Antimon und Wismut bestehenden Gruppe, $R^{15}$ ein Niederalkylrest mit 1 bis etwa 4 Kohlenstoffatomen ist und x 2 bis 5 ist, in Abhängigkeit von der Wertigkeit des Metallatoms;

(b) Isolieren des N-substituierten Acrylamids aus dem Filtrat, welches den Katalysatorrückstand enthält; und

(c) Wiederholen der Stufe (a) mit dem Katalysatorrückstand als Katalysatorverbindung.

**13.** Verfahren nach Anspruch 12, worin zusätzliche Mengen Katalysator zu dem Katalysatorrückstand gegeben werden.

## Revendications

**1.** Procédé pour la synthèse catalysée d'acrylamides N-substitués, caractérisé par la mise en réaction, à une température s'étalant de 70° à 150°C, d'un ester alkylique de l'acide acrylique ou de l'acide méthacrylique avec une amine aliphatique ou aromatique en présence d'un inhibiteur de polymérisation contenant un groupe nitroxyle et en présence de 0,05% à 20%, basé sur le poids de l'amine, d'un composé de catalyseur choisi parmi le groupe comprenant un oxyde d'alkylétain, un alcoxyde de trialkylétain, un oxyde de dialkylétain ou un alcoxyde métallique répondant à la formule :

$$M(OR^{15})_x$$

dans laquelle M est un métal choisi parmi le groupe comprenant le lanthane, le niobium, le tantale, le cuivre, le zinc, l'étain, le plomb, l'antimoine et le bismuth, $R^{15}$ représente un groupe alkyle inférieur contenant de 1 à environ 4 atomes de carbone et x représente une valeur de 2 à 5 en fonction de la valence de l'atome de métal, ou d'un résidu dudit composé de catalyseur, dans lequel l'inhibiteur de polymérisation est choisi parmi le groupe comprenant :

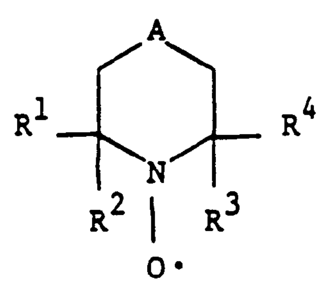

(I),

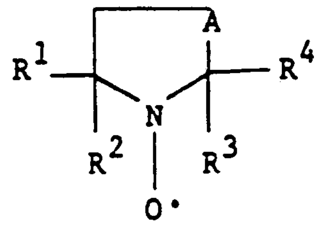

(II),

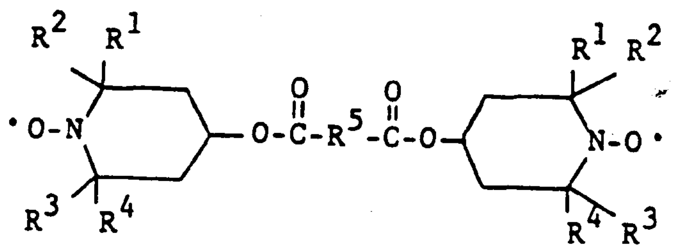

(III),

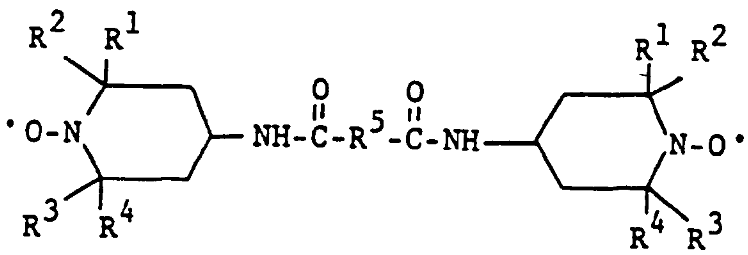

(IV),

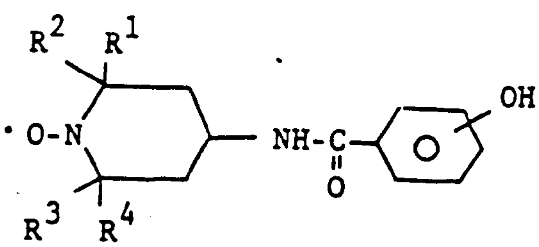

(V),

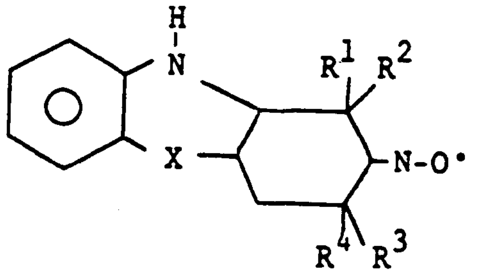

(VI),

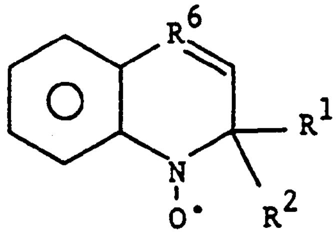

(VII),

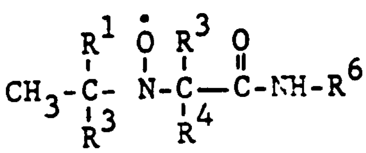

(VIII),

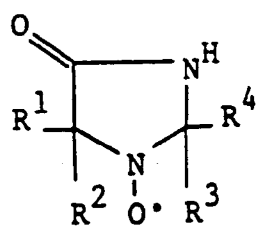

(IX),

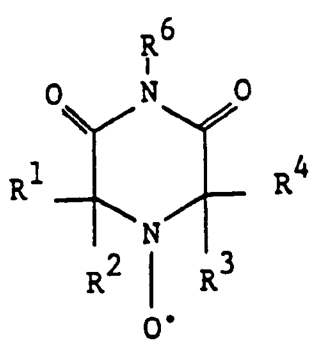

(X),

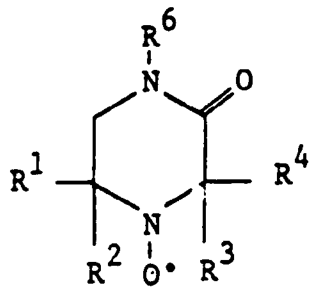

(XI),

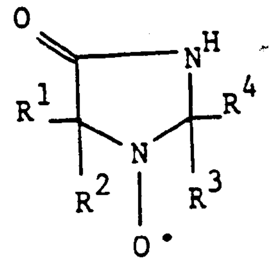

(XII),

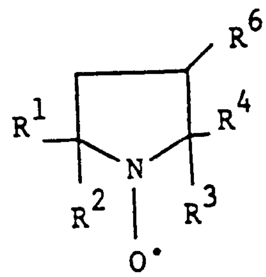

(XIII),

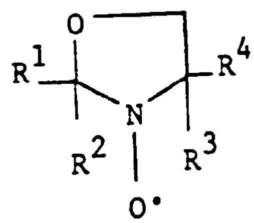

(XIV),

et

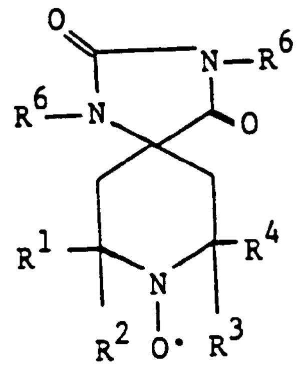

(XV)

2. Procédé selon la revendication 1, caractérisé en ce que l'inhibiteur de polymérisation est choisi parmi le groupe comprenant ceux de formule II et de formule III dans lesquelles $R^1$, $R^2$, $R^3$ et $R^4$ représentent, de manière indépendante, un radical alkyle linéaire contenant un atome de carbone; $R^5$ représente un radical alkylène contenant 8 atomes de carbone ou encore un radical arylène contenant 6 atomes de carbone, et A représente $-CH_2-$ ou -CH-OH.

3. Procédé selon la revendication 1, caractérisé en ce que ledit ester acrylique ou méthacrylique répond à la formule :

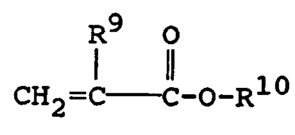

dans laquelle $R^9$ représente un radical d'hydrogène ou un radical méthyle et $R^{10}$ représente un radical alkyle contenant d'environ 1 à 4 atomes de carbone.

4. Procédé selon la revendication 1, caractérisé en ce que ladite amine répond à la formule :

$$HNR^{11}R^{12}$$

dans laquelle $R^{11}$ représente un radical d'hydrogène ou un radical alkyle contenant d'environ 1 à 4 atomes de carbone et $R^{12}$ représente un radical alkyle contenant d'environ 1 à environ 20 atomes de carbone, un radical alkaryle contenant d'environ 7 à 20 atomes de carbone, un radical aralkyle contenant d'environ 7 à environ 20 atomes de carbone ou encore un radical alcoxyalkyle contenant d'environ 2 à environ 20 atomes de carbone, ou :

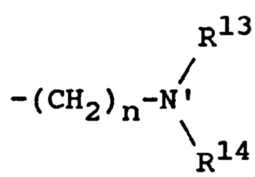

où n est un entier de 2 à 6, et $R^{13}$ et $R^{14}$ représentent, de manière indépendante, des groupes alkyle contenant d'environ 1 à 4 atomes de carbone ou bien $R^{13}$ et $R^{14}$ pris de manière conjointe sont combinés à l'atome N' pour former un groupe à noyau hétérocyclique choisi parmi le groupe comprenant des groupes à noyau morpholine, pyrrolidine ou pipéridine.

5. Procédé selon la revendication 1, caractérisé en ce que le rapport molaire entre ladite amine et ledit ester alkylique de l'acide acrylique ou de l'acide méthacrylique se situe d'environ 1:1 à environ 1:100.

6. Procédé selon la revendication 1, caractérisé en ce que ledit inhibiteur de polymérisation est présent en une quantité se situant dans l'intervalle d'environ 10 ppm à environ 1.500 ppm, basé sur le poids de l'ester.

7. Procédé selon la revendication 1, caractérisé en ce que ledit catalyseur est un oxyde de dialkylétain dans lequel chaque radical alkyle représente, de manière indépendante, un radical alkyle contenant d'environ 1 à 12 atomes de carbone.

8. Procédé selon la revendication 1, caractérisé en ce que ledit oxyde de dialkylétain est l'oxyde de dibutylétain.

9. Procédé selon la revendication 1, caractérisé en ce que ledit catalyseur est un alcoxyde d'alkylétain, un alcoxyde de dialkylétain ou un alcoxyde de trialkylétain.

10. Procédé selon la revendication 9, caractérisé en ce que ledit catalyseur est choisi parmi le groupe comprenant le triméthoxyde de butylétain, le triéthoxyde de butylétain, le diméthoxyde de dibutylétain, le diéthoxyde de dibutylétain, le diisopropoxyde de dibutylétain, le t-butoxyde de dibutylétain, le diméthoxyde de dioctylétain, le diéthoxyde de dioctylétain, le méthoxyde de tributylétain, l'éthoxyde de tributylétain, le méthoxyde de triéthylétain, le méthoxyde de triméthylétain et l'éthoxyde de trioctylétain.

11. Procédé selon la revendication 1, caractérisé en ce que ledit procédé est mis en oeuvre en présence d'un solvant choisi parmi le groupe comprenant le toluène, le xylène, le mésitylène et le décahydronaphtalène.

12. Procédé selon la revendication 1, caractérisé par :
(a) la mise en réaction, à une température qui se situe dans l'intervalle de 70°C à 150°C, d'un ester alkylique de l'acide acrylique ou de l'acide méthacrylique répondant à la formule :

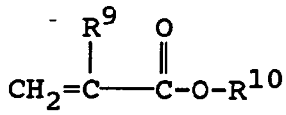

avec une amine de formule :

$HNR^{11}R^{12}$

en présence d'un inhibiteur de polymérisation choisi parmi le groupe comprenant :

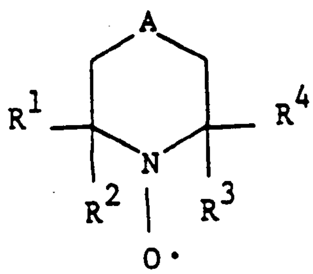

(I),

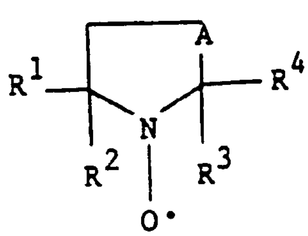

(II),

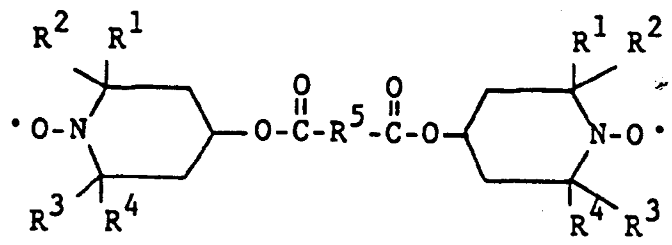

(III),

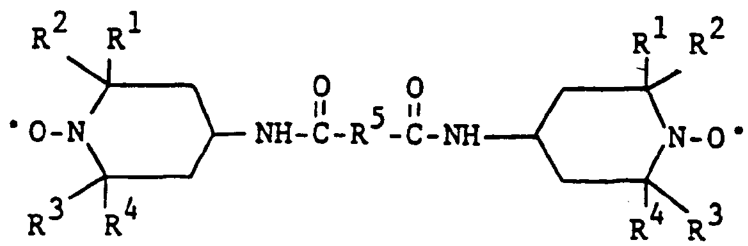

(IV),

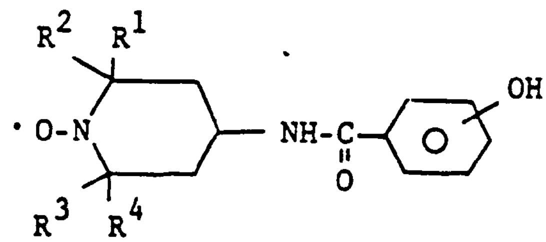

(V),

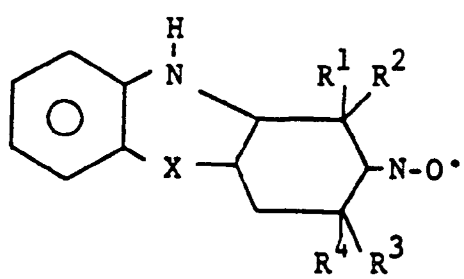

(VI),

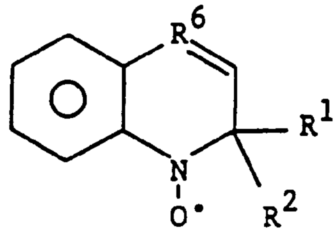

(VII),

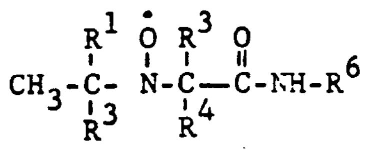

(VIII),

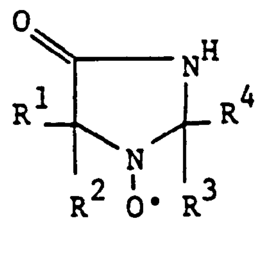

(IX),

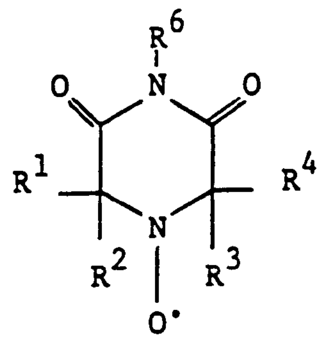

(X),

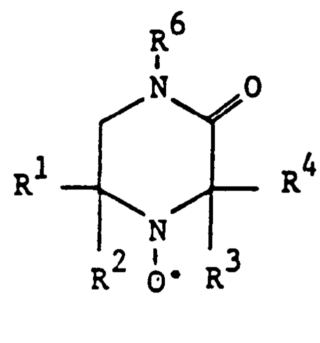

(XI),

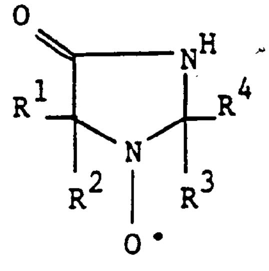

(XII),

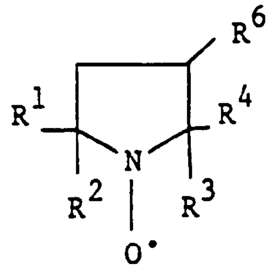

(XIII),

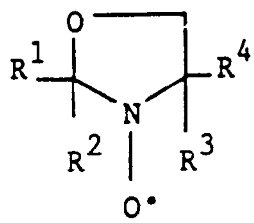

(XIV),

et

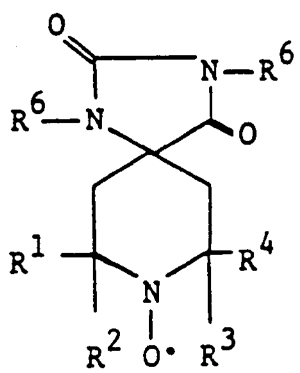

(XV)

en présence de 0, 05% à 20%, basé sur le poids de l'amine, d'un composé de catalyseur choisi parmi le groupe comprenant un alcoxyde d'alkylétain, un alcoxyde de trialkylétain, un oxyde de dialkylétain ou un alcoxyde métallique répondant à la formule :

$$M(OR^{15})_x$$

où $R^1$, $R^2$, $R^3$ et $R^4$ représentent, de manière indépendante, des radicaux alkyle linéaires ou exempts de ramification en position alpha, contenant de 1 à 9 atomes de carbone, ou bien un des radicaux $R^1$ et $R^2$ ou les deux ou un des radicaux $R^3$ et $R^4$ ou les deux forment un noyau cycloalkyle contenant de 5 à 7 atomes de carbone, y compris l'atome de carbone dans l'hétérocycle; où A représente

$-CH_2-$, $-C=O$,

$-CH-OR^8-$,

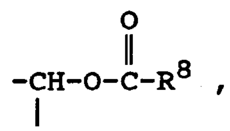

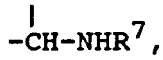

,

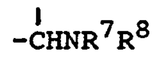

où $R^7$ et $R^8$ représentent, de manière indépendante, des radicaux d'hydrogène, des radicaux alkyle contenant de 1 à 18 atomes de carbone, des radicaux aryle contenant de 6 à 14 atomes de carbone ou des radicaux aralkyle contenant de 7 à 15 atomes de carbone; où $R^5$ représente un radical alkylène contenant de 5 à 10 atomes de carbone, un radical arylène contenant de 6 à 14 atomes de carbone ou encore un radical aralkylène contenant de 7 à 15 atomes de carbone; où $R^6$ représente, de manière indépendante, un radical alkyle contenant de 1 à 18 atomes de carbone, un radical aryle contenant de 6 à 14 atomes de carbone ou un radical aralkyle, et où X représente NH, S ou O; où $R^9$ représente un radical d'hydrogène ou un radical méthyle et $R^{10}$ représente un radical alkyle contenant d'environ 1 à 4 atomes de carbone, où $R^{11}$ représente un radical d'hydrogène ou un radical alkyle contenant d'environ 1 à 4 atomes de carbone et $R^{12}$ représente un radical alkyle contenant d'environ 1 à environ 20 atomes de carbone, un radical alkaryle contenant d'environ 7 à environ 20 atomes de carbone, un radical aralkyle contenant d'environ 7 à 20 atomes de carbone, un radical alcoxyalkyle contenant d'environ 2 à environ 20 atomes de carbone, ou :

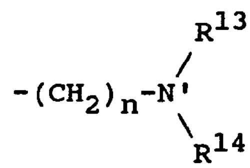

où n est un entier de 2 à 6, et $R^{13}$ et $R^{14}$ représentent, de manière indépendante, des groupes alkyle contenant d'environ 1 à 4 atomes de carbone ou bien $R^{13}$ et $R^{14}$, pris de manière conjointe, sont combinés à l'atome N' pour former un groupe à noyau hétérocyclique choisi parmi le groupe comprenant des groupes à noyau morpholine, pyrrolidine et pipéridine; où M est un métal choisi parmi le groupe comprenant le lanthane, le niobium, le tantale, le cuivre, le zinc, l'étain, le plomb, l'antimoine et le bismuth, $R^{15}$ représente un radical alkyle inférieur contenant de 1 à environ 4 atomes de carbone et x représente une valeur de 2 à 5 en fonction de la valence de l'atome de métal;

(b) isoler du filtrat contenant le résidu de catalyseur l'acrylamide N-substitué; et

(c) répéter l'étape (a) avec le résidu de catalyseur comme composé de catalyseur.

**13.** Procédé selon la revendication 12, dans lequel des quantités supplémentaires de catalyseur sont ajoutées au résidu de catalyseur.